# EUROPEAN PATENT SPECIFICATION

(11) **EP 1 432 461 B1**
(45) Date of publication and mention of the grant of the patent: **15.11.2006**
(21) Application number: 02763106.8
(22) Date of filing: 30.09.2002
(51) Int. Cl.: A61L 24/02, A61L 27/38

(54) **INJECTABLE CALCIUM SALT BONE FILLER COMPRISING CELLS**
INJIZIERBARES KNOCHENERSATZMATERIAL AUF BASIS VON CALCIUMSALZEN UND ZELLEN
CHARGE OSSEUSE DE SEL DE CALCIUM INJECTABLE COMPORTANT DES CELLULES

(30) Priority: 02.10.2001 EP 01203746
(43) Date of publication of application: 30.06.2004
(73) Proprietor: IsoTis N.V., 3723 MB Bilthoven (NL)
(72) Inventor: DE BRUIJN, Joost, Dick, NL-3817 JL Amersfoort (NL); FISCHER, Elisabeth, Maria, NL-1211 DE Hilversum (NL); LAYROLLE, Pierre, Jean, François, F-72000 Le Mans (FR)
(74) Representative: Winckels, Johannes Hubertus F.
(86) International application number: PCT/NL2002/000633
(87) International publication number: WO 2003/028779

(56) References cited:
- WO-A-00/07639
- US-A1- 2001 014 475
- US-B1- 6 287 341

## Description

The invention relates to the field of reconstructive surgery, and in particular to the repair of osseous defects in a patient.

Successful closure of bone defects remains a major concern to reconstructive surgeons. While most often secondary to trauma, bone loss can also arise from congenital disorders, neoplasms, and infections. A wide variety of materials have been employed to repair osseous defects, including autogenous cells, allogeneic tissues, and alloplastic materials. This variety of approaches attests to the absence of an optimal method for restoring bone integrity, especially in the presence of a sizable defect.

Aside from the selection of a suitable material for repair of an osseous defect, the reconstructive surgeon is also faced with the problem of accessibility. In order to be able to insert a reconstructive material at the site of the defect, it is often necessary to make considerable wounds, causing trauma and discomfort to the patient, since typically the location of the osseous defect is inside the body in the bone structure of the patient.

Conventional constructs used to repair osseous defects have a rigid, inflexible structure, as they must be able to take over the supporting tasks of living bone tissue. Also, they are of a size dependent on the size of the osseous defect, as the complete defect should preferably be repaired in one surgical operation. Hence, the larger the defect that is in need of repair, the greater the opening of the wound must be in order to be able to insert the construct into the defect.

In view of these circumstances, there is a need for a material that is suitable to function in the repair of bone tissue which has a flexibility that allows its introduction into the patient's body through a small wound opening, but nevertheless has such mechanical properties that enables it to assist in the supporting function of bone tissue, and preferably is ultimately converted into actual bone tissue.

US patent 6,129,761 discloses an injectable hydrogel composition comprising a hydrogel based on hyaluronic acid, a synthetically modified alginate, or another crosslinkable polymer capable of forming a hydrogel, and dissociated cells, such as bone cells, muscle cells, fibroblasts or organ cells. The composition is specifically intended for cartilage or organ repair.

The international patent application 95/21634 discloses a biomaterial for the resorption/substitution of supporting tissue, tooth substance, bony tissue or osteoarticular tissue. The composition is injectable and comprises an inorganic phase of calcium phosphate particles, and an aqueous solution of a cellulose-derived polymer. The calcium phosphate particles need to be either a mixture of tricalcium phosphate β and hydroxyapatite in a ratio of 20/80-70/30, or calcium-titanium-phosphate.

US patent 6,287,341 discloses a method for repairing an osseous defect wherein two calcium phosphates are mixed with a physiological liquid to provide a paste or putty which is applied to the osseous defect to harden at the implant site. The hardening occurs as a result of a reaction between the two calcium phosphates. It is mentioned that the paste or putty may comprise live cells, such as osteoblasts, osteoclasts, chondrocytes, osteocytes or fibroblasts. These cells, however, are not expected to be able to withstand the harsh conditions during the hardening of the paste or putty.

The international patent application 00/07639 discloses bone precursor compositions. A calcium cement is mentioned for being suitable for injection into a bone defect. The cement is based on monobasic calcium phosphate monohydrate and β-tricalcium phosphate, and may further comprise a biopolymer foam, collagen, an extracellular matrix component, a therapeutic agent, a biopolymer fibre, or live cells. After injection, the calcium cement require setting, which is likely to be harmful to any living cells present.

It is an objective of the present invention to provide a bone filler which can be used for tissue repair, which bone filler comprises cells, wherein the risk of harm to the cells (e.g. due to setting of a calcium phosphate phase) is substantially avoided. The objective bone filler should have such properties that it can be easily processed and be injected into an osseous defect in a patient through the needle of a syringe under sterile conditions. It is further desired that the cells will not be substantially harmed by being injected through for instance a syringe. Other objects and advantages of the invention will become clear from the following description.

In accordance with the invention, an injectable bone filler is provided, which bone filler comprises calcium salt particles, an organic binder having an affinity for the calcium salt, cells chosen from the group of stem cells, osteogenic cells, and osteoprogenitor cells, and a pharmaceutically acceptable buffer.

A bone filler according to the invention is injectable, which means that it can be administered to the site of an osseous defect through injection. To this end, it is preferred that a syringe is employed. The bone filler has such flexibility that it can pass through the needle of a syringe. This has as a great advantage that only a very small wound needs to be made in order to introduce the filler at the desired location, which spares the patient a considerable discomfort and possible trauma.

Further, the presence of calcium salt particles in the bone filler allows for *de novo* bone formation *in vivo.* As a result, the filler is ultimately converted into autologous bone tissue and can assist in the supporting function of the bone in an early stage. Also, it was found that the calcium salt particles may function as a kind of seeding crystals *in vivo* on which additional calcium salt is deposited. Accordingly, the bone filler hardens and provides strength soon after implantation.

Surprisingly, it has further been found that living cells can be incorporated into the formulation of a bone filler according to the invention in such a manner that the bone filler can be injected without substantially negatively affecting the viability of the cells. In fact, the presence of the cells in the bone filler have a significant positive impact on the rate at which bone formation occurs *in vivo* after administration of the bone filler (in)to an osseous defect.

As mentioned above, a bone filler according to the invention comprises calcium salt particles. Dependent on the location of an osseous defect that is to be repaired with the filler, the skilled person can suitably select a calcium salt. Possible choices are for instance monetite, brushite, (CaHPO₄), calcium pyrophosphate, and calcium carbonate. Preferred is the use of calcium phosphate salts, in particular hydroxyapatite, β-calcium phosphate, and combinations thereof, such as in a mass ratio of 60/40. All of these materials occur naturally in living bone and are consequently readily accepted by a living organism. Particularly good results have been achieved using hydroxyapatite.

An important parameter of the calcium salt particles was found to be their particle size. Preferably, the particles have a diameter of from 100 to 600 µm, more preferably of from 200 to 400 µm. As is also shown in the appended examples, a relationship was surprisingly found between the size of the calcium salt particles and rate and extent of bone formation induced *in vivo.*

Calcium salt particles of the desired size can conveniently be prepared by crushing calcium salt and sieving at the right mesh size. It is preferred that a sintered calcium salt is used, which is optionally water tumbled before sintering to obtain a dense material. It is preferred that dense and smooth calcium salt particles are employed, as this significantly reduces the risk of inflammation *in vivo*.

Another important substance present in a bone filler according to the invention is the organic binder. The binder should have sufficient affinity for the calcium salt to allow the formation of a homogeneous paste to form the injectable bone filler. Further, it will be understood that the binder should be of a material that is acceptable for introduction into a living organism. Preferably, the binder is biodegradable so that it disappears once the deposition of calcium salt and/or the bone formation has taken place to a sufficient extent to take over the function of living bone.

It is furthermore desired that the binder contributes to the viscosity of the bone filler. It serves on the one hand to keep the calcium salt particles together as to form a paste of sufficient integrity, and on the other hand to impart sufficient flexibility to the bone filler to allow for its administration through the needle of a syringe.

Suitable examples of materials that can be used as the organic binder in a bone filler according to the invention include alginates, dextrans, cellulose, derivatives of cellulose, plasma (blood plasma), biogenic binders, hyaluronic acid, and combinations thereof. Specific examples are sodium alginate, sodium carboxymethyl cellulose, dextran, fibrin glue, and transglutaminase. It is preferred to use sodium alginate as it was found that this binders allows for a very convenient formulation of the bone filler.

Dependent on the nature of the binder chosen, it is preferably present in a bone filler according to the invention in an amount ranging from 0.5 to 10 wt.%, more preferably from 3 to 7 wt.%, based on the weight of the bone filler.

Suitable cells that may be incorporated are stem cells, osteogenic cells, and osteoprogenitor cells. It is preferred that the cells that are incorporated into the bone filler are obtained through a biopsy from the patient to which the bone filler is ultimately to be administered, i.e. that autologous cells are used.

In order to assist in the formulation of a bone filler according to the invention, it is usually preferred to use and incorporate a buffer. The buffer can also serve to ensure that the osmolarity of a bone filler according to the invention is similar to the osmolarity in the surroundings of the osseous defect into which the bone filler is to be injected, thereby avoiding an undesired impact of the filler on living tissue at the site of implantation. Although in principle any liquid that is sufficiently pharmaceutically acceptable can be used, it is preferred that a saline solution essentially not causing osmotic pressure to cells (usually around 8 g/L) and comprising a biocompatible buffer (preferably at a pH around 7.4) is employed. Especially preferred is the use of phosphate buffer saline (PBS) as buffer.

The amount of buffer used will depend on the viscosity of the chosen binder and the desired viscosity of the bone filler. Generally, the bone filler will be formulated to have a solids content of 30-70 wt.%, preferably 40-60 wt.%.

In order for a bone filler according to the invention to pass through a needle of a syringe without great difficulty, its Brookfield viscosity will generally lie between 30,000 and 100,000 centipoises.

In the preparation of a bone filler according to the invention, it has proven to be of advantage to first prepare a gel of the organic binder and the buffer. To this end, the binder is mixed with or dissolved in the buffer. Preferably, and depending on the binder, care is taken during mixing that the binder does not form agglomerates. To the prepared gel, the calcium salt particles can be added and they can be mixed to form a homogeneous paste, being the objective bone filler.

In a preferred embodiment, cells are seeded onto the calcium salt particles before they are added to the gel formed by the organic binder and the buffer. It is also possible to introduce the cells after the calcium salt particles, the organic binder and the buffer are brought together. In the latter embodiment, it is possible that the cells actually adhere to the calcium salt particles prior to injection of the filler into a patient, but it is also possible that they will be part of the injectable bone filler as a separate component. If the cells adhere to the calcium salt particles, it can be said that the particles are coated with cells.

The seeding of the cells to the calcium salt particles can be carried out in any conventional manner. Preferably, the cells are cultured for one or more passages before the calcium salt particles carrying the cells are formulated together with the gel formed by the organic binder and buffer. The culturing is preferably performed under dynamic conditions, e.g. as described in European patent application 1002 859, in order to retain sufficient fluidity. During the culturing, proliferation and differentiation may occur, as desired. Often abundant extracellular matrix is produced which might cluster the cells together. Any suitable culture medium may be employed for the culturing, e.g. a culture medium as disclosed in WO 01/48147. In a preferred embodiment, this culture medium may be mixed to a desired extent with the buffer used in the formulation of a bone filler according to the invention.

It is preferred that an injectable bone filler according to the invention further comprises an osteoinductive factor. This factor will typically be incorporated in an amount in the range of 0.01 to 3 wt.%, based on the weight of the bone filler. Examples of suitable osteoinductive factors include growth factors such as BMP.

It has further been found advantageous to incorporate an angiogenic factor into the bone filler. An angiogenic factor may be used both in a bone filler that does not comprise cells, and in a bone filler that does. An angiogenic factor will typically be incorporated in an amount in the range of 0.01 to 3 wt.%, based on the weight of the bone filler. Examples of suitable osteoinductive factors include growth factors such as FGF, VEGF, and PDGF.

It will be understood that the invention also encompasses a syringe having a needle and a reservoir wherein the reservoir contains a bone filler as described above. It will furthermore be understood that the syringe is to be kept under sterile conditions.

Of course, the invention further also encompasses the use of a bone filler as described above in the repair of osseous defects, wherein the bone filler is introduced into the defect by injection.

The invention will now be further elucidated by the following, non-restrictive examples.

### Example I

Fourth passage goat bone marrow cells were seeded onto densely sintered hydroxyapatite granules with a size of 212 to 300 micrometers, in a concentration of 200,000 cells per 200 milligram of hydroxyapatite. The cells were grown on the scaffold for 7 days in osteogenic culture medium comprising alpha-MEM, 15% foetal bovine serum, 0.2mM ascorbic acid-2-phosphate, 2mM L-glutamine, 10nM dexamethasone, 10mM beta-glycerophosphate and penicillin/streptomycin. The cell-coated granulate was subsequently mixed with a 3% alginate gel in PBS (sodium salt alginic acid, high viscosity, Sigma A7128) in a ratio of 58% alginate gel and 42% cell-coated hydroxyapatite (w/w). This paste was then subcutaneously implanted in nude mice (HsdCP:NMRI-nu, Harlan). After 4 weeks, the samples were retrieved and examined histologically.

A comparative study was performed wherein a paste was implanted, which was obtained by combining the three components hydroxyapatite granules, a cell suspension and an alginate gel (as described above) just prior to implantation, after which histology was performed 4 weeks post-operatively.

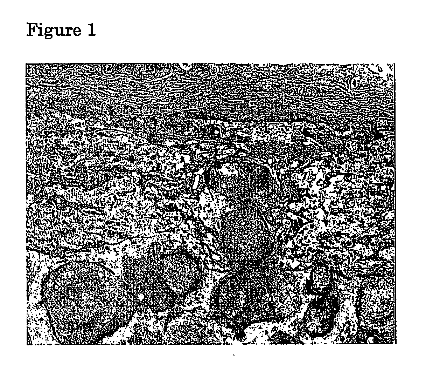

With both experiments, histological evaluation revealed that a fibrous tissue surrounded the implanted material paste. No signs of an inflammatory reaction could be observed, nor could histological differences be observed between implantation of the paste in mice or rats. At the periphery of the implant, early stages of tissue ingrowth and blood vessel formation were seen. Figure 1 shows the tissue reaction around hydroxyapatite granulate mixed with alginate gel after 4 weeks of implantation in Fischer rats. Note the fibrous tissue encapsulation and the absence of an inflammatory reaction.

From this study, it can be concluded that hydroxyapatite granulate, coated or combined with bone marrow cells and mixed with an injectable carrier such as alginate, results in a biocompatible injectable bone filler.

### Example II

PBS and algenic acid were mixed with a Braun multimixer or a blender. It is not preferred to do this with a normal mixer, because the alginate may agglomerate. Mix for 30 seconds with blender then 5 seconds by hand to prevent the alginate agglomerates sticking at the wall, then mix for another 30 sec. Mixing will cause a lot of air bubbles in the resulting gel. It is possible to suck these out of the gel with a vacuum furnace/pump twice for 5 seconds. Because of this, some water will vaporise. The amount of lost is ±0.16% (this depends of course on the surface where the vaporization can take place).

In the gel thus obtained, hydroxyapatite particles (HA) were introduced through mixing. Different amounts of hydroxyapatite particles, as well as different sizes of hydroxyapatite apatite were studied and evaluated for injectability.

The injectability tests were performed with a Geniaplex syringe of 50 ml from the company Genia. If not mentioned different the outlet is a threated luer hub (code 109302, Genia). The syringe was fixed and the piston was connected with the loadcell of the tensile bench. The speed of testing is 75 mm/min. This speed was chosen, because it is more or less a normal speed of manual-injection. Every result is the average of a triple test. The Max (average) is the average of the 5 maximum tensile force points.

The following tables show the results achieved.

**Table I: amount of HA (particle size 212-300 µm, dense) v force for injection through needle of 2.2 mm diameter**

| Amount of HA in gel (wt.%) | Force needed for injection (N) |
|---|---|
| 35 | 15.4 |
| 43 | 21.12 |
| 48 | 57.3 |

**Table II: particle size of HA (in identical amounts) v force for injection through needle of 2.2 mm diameter**

| Particle size of HA (µm) | Surface of particles | Force needed for injection (N) |
|---|---|---|
| 212-300 | Rough | 70.5 |
| 212-300 | Dense/smooth | 29.8 |
| 300-500 | Dense/smooth | 28.1 |

**Table III: length of needle (diameter 2.2 mm) v force of injection**

| Length of needle (cm) | Force needed for injection (N) |
|---|---|
| 0 | 78.8 |
| 0.5 | 85.4 |
| 1 | 89.8 |
| 2 | 92 |
| 3.5 | 107.2 |
| 5 | 139.4 |

## Claims

1. Injectable bone filler comprising sintered calcium salt particles, an organic binder having an affinity for the calcium salt, cells chosen from the group of stem cells, osteogenic cells, and osteoprogenitor cells, and a pharmaceutically acceptable buffer.

2. Bone filler according to claim 1, wherein the particles are of a calcium salt are chosen from the group of calcium phosphates, monetite, brushite, (CaHPO₄), calcium pyrophosphate, calcium carbonate, and combinations thereof.

3. Bone filler according to claim 2, wherein the calcium salt is hydroxyapatite, β-calcium phosphate, and combinations thereof.

4. Bone filler according to claim 3, wherein the particles have a diameter in the range of 100 to 600 µm, preferably 200 to 400 µm.

5. Bone filler according to any of the preceding claims, wherein the binder is chosen from the group of alginates, dextrans, cellulose, cellulose derivates, biogenic binders, hyaluronic acid, and combinations thereof.

6. Bone filler according to claim 5, wherein the binder is chosen from the group of hyaluronic acid, sodium alginate, sodium carboxymethyl cellulose, dextran, fibrin glue, and transglutaminase.

7. Bone filler according to claim 6, wherein the binder is sodium alginate.

8. Bone filler according to any of the preceding claims, wherein the binder is present in an amount of from 0.5 to 10 wt.%, preferably from 3 to 7 wt.%, based on the weight of the bone filler.

9. Bone filler according to any of the preceding claims, wherein the buffer is phosphate buffer saline (PBS).

10. Bone filler according to any of the preceding claims having a solids content of 30-70, preferably 40-60 wt.%.

11. Bone filler according to any of the preceding claims having a viscosity between 30,000 and 100,000 centipoises.

12. Bone filler according to any of the preceding claims further comprising an angiogenic factor.

13. Bone filler according to any of the preceding claims, wherein the cells are present in seeded form onto the calcium salt particles.

14. Bone filler according to any of the preceding claims further comprising an osteoinductive factor.

15. Syringe comprising a needle and a reservoir, which reservoir comprises an injectable bone filler according to any of the preceding claims.

16. Syringe according to claim 15, wherein the needle has a length between 5 and 20 mm, and a diameter between 2 and 5 mm.

17. Method for preparing an injectable bone filler according to any of the claims 1-14, comprising mixing the binder and the buffer to prepare a gel, adding the calcium salt particles to the gel, and homogenizing to obtain the bone filler.

18. Method according to claim 17, in which cells are seeded onto the calcium salt particles before they are added to the gel, or wherein cells are introduced after combining calcium salt particles and the gel, the cells being chosen from the group of stem cells, osteogenic cells, and osteoprogenitor cells.

19. Use of an injectable bone filler according to any of the claims 1-14 for the manufacture of a medicament for repairing an osseous defect by injecting said filler into the defect..

## Patentansprüche

1. Injizierbares Knochenersatzmaterial, umfassend gesinterte Calciumsalzteilchen, ein organisches Bindemittel mit Affinität für das Calciumsalz, aus der Gruppe Stammzellen, osteogene Zellen und Knochenvorläuferzellen ausgewählte Zellen und einen pharmazeutisch verträglichen Puffer.

2. Knochenersatzmaterial nach Anspruch 1, bei dem die Teilchen eines Calciumsalzes aus der Gruppe Calciumphosphate, Monetit, Brushit (CaHPO₄), Calciumpyrophosphat, Calciumcarbonat und Kombinationen davon ausgewählt werden.

3. Knochenersatzmaterial nach Anspruch 2, bei dem das Calciumsalz Hydroxyapatit, β-Calciumphosphat und eine Kombination davon ist.

4. Knochenersatzmaterial nach Anspruch 3, bei dem die Teilchen einen Durchmesser im Bereich von 100 bis 600 µm, vorzugsweise 200 bis 400 µm haben.

5. Knochenersatzmaterial nach einem der vorstehenden Ansprüche, bei dem das Bindemittel aus der Gruppe Alginate, Dextrane, Cellulose, Cellulosederivate, biogene Bindemittel, Hyaluronsäure und Kombinationen davon ausgewählt wird.

6. Knochenersatzmaterial nach Anspruch 5, bei dem das Bindemittel aus der Gruppe Hyaluronsäure, Natriumalginat, Natriumcarboxymethylcellulose, Dextran, Fibrinkleber und Transglutaminase ausgewählt wird.

7. Knochenersatzmaterial nach Anspruch 6, bei dem das Bindemittel Natriumalginat ist.

8. Knochenersatzmaterial nach einem der vorstehenden Ansprüche, bei dem das Bindemittel bezogen auf das Gewicht des Knochenersatzmaterials in einer Menge von 0,5 bis 10 Gew.-%, vorzugsweise 3 bis 7 Gew.-% vorliegt.

9. Knochenersatzmaterial nach einem der vorstehenden Ansprüche, bei dem der Puffer Phosphatpuffersalzlösung (PBS) ist.

10. Knochenersatzmaterial nach einem der vorstehenden Ansprüche mit einem Feststoffgehalt von 30 bis 70, vorzugsweise 40 bis 60 Gew.-%.

11. Knochenersatzmaterial nach einem der vorstehenden Ansprüche mit einer Viskosität zwischen 30.000 und 100.000 cPs.

12. Knochenersatzmaterial nach einem der vorstehenden Ansprüche, das außerdem einen angiogenen Faktor umfasst.

13. Knochenersatzmaterial nach einem der vorstehenden Ansprüche, bei dem die Zellen in auf die Calciumsalzteilchen geimpfter Form vorliegen.

14. Knochenersatzmaterial nach einem der vorstehenden Ansprüche, das außerdem einen das Knochenwachstum induzierenden Faktor umfasst.

15. Spritze, umfassend eine Nadel und ein Reservoir, wobei das Reservoir ein injizierbares Knochenersatzmaterial nach einem der vorstehenden Ansprüche umfasst.

16. Spritze nach Anspruch 15, bei der die Nadel eine Länge zwischen 5 und 20 mm und einen Durchmesser zwischen 2 und 5 mm hat.

17. Verfahren zur Herstellung eines injizierbaren Knochenersatzmaterials nach einem der Ansprüche 1 bis 14, umfassend das Mischen des Bindemittels und des Puffers, um ein Gel herzustellen, die Zugabe der Calciumsalzteilchen zu dem Gel und das Homogenisieren, um das Knochenersatzmaterial zu erhalten.

18. Verfahren nach Anspruch 17, bei dem Zellen auf die Calciumsalzteilchen geimpft werden, ehe diese dem Gel zugesetzt werden, oder bei dem die Zellen nach dem Kombinieren der Calciumsalzteilchen und des Gels eingeführt werden, wobei die Zellen aus der Gruppe Stammzellen, osteogene Zellen und Knochenvorläuferzellen ausgewählt werden.

19. Verwendung eines injizierbaren Knochenersatzmaterials nach einem der Ansprüche 1 bis 14 zur Herstellung eines Medikaments zur Reparatur eines Knochendefekts durch Injizieren des Ersatzmaterials in den Defekt.

## Revendications

1. Charge osseuse injectable comprenant des particules de sel de calcium fritté, un liant organique ayant une affinité pour le sel de calcium, des cellules choisies dans le groupes des cellules souches, des cellules ostéogéniques et des cellules ostéoprogénitrices, et un tampon acceptable du point de vue pharmaceutique.

2. Charge osseuse selon la revendication 1, dans laquelle les particules d'un sel de calcium sont choisies dans le groupe consistant en phosphates de calcium, monétite, brushite, (CaHPO₄), pyrophosphate de calcium, carbonate de calcium et combinaisons de ceux-ci.

3. Charge osseuse selon la revendication 2, dans laquelle le sel de calcium est l'hydroxyapatite, le β-phosphate de calcium et leurs combinaisons.

4. Charge osseuse selon la revendication 3, dans laquelle les particules ont un diamètre compris dans la gamme de 100 à 600 µm, de préférence de 200 à 400 µm.

5. Charge osseuse selon l'une quelconque des revendications précédentes, dans laquelle le liant est choisi dans le groupe consistant en alginates, dextranes, cellulose, dérivés cellulosiques, liants biogéniques, acide hyaluronique et combinaisons de ceux-ci.

6. Charge osseuse selon la revendication 5, dans laquelle le liant est choisi dans le groupe consistant en acide hyaluronique, alginate de sodium, carboxyméthyl cellulose sodique, dextrane, colle de fibrine et transglutaminase.

7. Charge osseuse selon la revendication 6, dans laquelle le liant est l'alginate de sodium.

8. Charge osseuse selon l'une quelconque des revendications précédentes, dans laquelle le liant est présent en une quantité de 0,5 à 10% en poids, de préférence de 3 à 7% en poids par rapport au poids de la charge osseuse.

9. Charge osseuse selon l'une quelconque des revendications précédentes, dans laquelle le tampon est une solution saline au tampon phosphate (PBS).

10. Charge osseuse selon l'une quelconque des revendications précédentes, ayant une teneur en solides de 30 à 70, de préférence de 40 à 60% en poids.

11. Charge osseuse selon l'une quelconque des revendications précédentes, ayant une viscosité de 30.000 à 100.000 centipoises.

12. Charge osseuse selon l'une quelconque des revendications précédentes, comprenant en outre un facteur angiogénique.

13. Charge osseuse selon l'une quelconque des revendications précédentes, dans laquelle les cellules sont présentes sur les particules de sel de calcium sous une forme de germes.

14. Charge osseuse selon l'une quelconque des revendications précédentes, comprenant de plus un facteur ostéoinducteur.

15. Seringue comprenant une aiguille et un réservoir, lequel réservoir comprend une charge osseuse selon l'une quelconque des revendications précédentes.

16. Seringue selon la revendication 15, dans laquelle l'aiguille a une longueur de 5 à 20 mm et un diamètre de 2 à 5 mm.

17. Procédé de préparation d'une charge osseuse injectable selon l'une quelconque des revendications 1 à 14, comprenant le mélange du liant et du tampon pour préparer un gel, l'addition des particules de sel de calcium au gel, et l'homogénéisation pour obtenir la charge osseuse.

18. Procédé selon la revendication 17, dans lequel les cellules sont ensemencées sur les particules de sel de calcium avant d'être ajoutées au gel, ou dans lequel des cellules sont introduites après combinaison des particules de sel de calcium et du gel, les cellules étant choisies dans le groupe des cellules souches, des cellules ostéogéniques et des cellules ostéoprogénitrices.

19. Utilisation d'une charge osseuse injectable selon l'une quelconque des revendications 1 à 14 pour la fabrication d'un médicament destiné à la réparation d'un défaut osseux par injection de ladite charge dans le défaut.
